# EUROPEAN PATENT APPLICATION

(11) **EP 1 129 729 A1**
(43) Date of publication of application: **05.09.2001**
(21) Application number: 00830161.6
(22) Date of filing: 02.03.2000
(51) Int. Cl.: A61L 9/014, A61L 9/04

(54) **Inert support made of absorbing material for the emission of fragrances**

(71) Applicant: Dueffe S.r.l., 60044 Fabriano (AN) (IT)
(72) Inventor: Lori, Giancarlo, Fabriano (AN) (IT)
(74) Representative: Baldi, Claudio

(57) **Abstract**

The present invention relates to an inert support made of absorbing material designed to be impregnated with fragrances, compositions, essences and perfumed aromas and capable of ensuring the optimal emission of such fragrances, essences, compositions or aromas in closed or open environments.

## Description

The present patent application relates to an inert support made of absorbing material to be used for the emission of fragrances, compositions, natural essences, synthetic essences, aromatic essences and aromas in closed or open environments.

As it is known, it is quite common to perfume closed environments in which people remain for long periods of time. This is true not only for domestic environments, but also for commercial places, public and private offices, as well as public and private transportation means.

A technique used to perfume such environments provides for using suitable sprays capable of emitting fragrances, compositions, natural or synthetic essences, aromatic essences and aromas. It is true, however, that these fragrances are generally quite volatile and, as such, inexorably subject to dissolve and lose their efficacy in short time.

Alternatively, the environments can be perfumed by means of a continuous emission of suitable fragrances, compositions, essences and aromas.

This technique provides for impregnating suitable supports made of inert materials - mostly fabric, plastics, paper or cardboard - with fragrances, both concentrated and diluted in various types of solvents.

According to this technique, the inert materials act like a sponge, meaning that they are capable of absorbing the fragrances and aromas due to the phenomenon known as "capillarity". Such phenomenon takes place when adhesion forces apply between a liquid (in which fragrances and aromas are dissolved) and a solid, that is the fibres of the material used for the inert support.

The progressive emission of fragrances from the inert supports is produced by the spontaneous migration of the molecules of the liquid fragrances towards the surrounding area.

The spontaneous migration is due to a known physical law according to which the atmosphere conditions of the environment always tend to reach the equilibrium. According to this law, the molecules of the perfumed liquid with an initial high concentration in the inert support tend to migrate to the surrounding air, free of fragrances, until the air has reached a concentration of molecules of perfumed liquid equal to the residual concentration of the molecules in the inert support.

A considerable disadvantage of the current technology is represented by the difficulty of providing a calibrated, regular and long emission of fragrances. In order to do it, the inert supports should be capable of slowing and diluting the release (or evaporation) of the fragrance molecules in time.

However, in the presence of traditional inert supports, it has been proved that the fragrance evaporation speed remains too high, since the capillary absorption of fragrances and aromas by the fibres of the inert support originates a very weak bond between the perfumed liquid molecules and the solid molecules of the support, characterised by limited adhesion forces.

More precisely, the traditional inert supports have at least two disadvantages, that is the rapid exhaustion of the perfumed charge and the excessive initial concentration of the perfumed emissions whose smell becomes unpleasant.

For these reasons inert supports with different characteristics (such as porous materials of ceramic type) have been introduced onto the market to provide a progressive emission of fragrances. However, the practical use has shown that this second type of inert supports excessively delay the emission of fragrances, almost inhibiting it.

In order to correct such defect, the inert supports work at a temperature higher than room temperature to induce a faster evaporation of fragrances.

This solution, however, has proved, quite unsatisfactory since the high temperature tends to induce a decomposition process of the fragrances, especially natural aromas.

In view of the above, the inert support according to the invention has been devised, which is capable of ensuring the optimal emission of fragrances, natural and synthetic essences, aromatic essences and aromas.

The inert support according to the invention is highly innovative with respect to the consolidated technique and absolutely original, since its working principle is the opposite of traditional supports.

While the operation of traditional supports is based on absorption, the operation of the new support according to the invention is based on the adsorption principle. According to it, the molecule of fragrances (both in the liquid and gaseous or steam state) are attracted and held not by physical adhesion forces of capillary type, but by electrical atomic forces thanks to the enormous contact surface existing inside the support.

To state that the new support has absorbing properties equals to say that he adhesion bond between the molecules of the perfumed liquid compound (volatile) and the support surface takes place on the surface of the internal pores of the absorbing material.

Thanks to this characteristic, during experimental testing, the new inert support according to the invention has ensured a prolonged, gradual and measured emission of fragrances, compositions, essences and aromas into the environment, that is without emission peaks or vacuums and without the need of providing heat.

For the practical realisation of the inert support according to the invention, two specific materials with absorbing properties have been identified for alternative use that are capable of providing extremely satisfactory results. This of course does not exclude the use of different absorbing materials.

The two preferred materials are:
- Activated carbon in the various levels of activity, expressed as absorption index of carbon tetrachloride (CTC) for drawn or extruded cylinders of any size and as iodine index for powder, granules or slices of any size.
- Molecular sieve or zeolite (in the various types of specificity measured in Angstrom, both of hydrophilic and hydrophobic type, in any spheroidal, cilindrical shape or powder).

As experts know, the term "activated carbon" is usually given to a highly porous material produced from carbonised raw materials and characterised by a considerable internal surface (from 400 to more than 1,600 m²/gram) and a considerable volume of pores (more than 30 cm³/gram).

On the other end, the term "molecular sieve or zeolite" is normally given to a highly porous crystalline material produced from alumina and sodium silicate and characterised by a considerable internal surface (more than 1,000 m²/gram) and a considerable volume of pores (more than 25 cm³/gram).

The various types of activated carbon and/or molecular sieve differ in the selectivity of molecular absorption of fragrances, compositions, essences and aromas, in the diameter and centesimal distribution of their internal pores, as well as in the intensity of the atomic bonds that vary from molecule to molecule (average diameter, chemical structure, polarity grade, etc.).

It can be stated that the ideal coupling between the type of support (activated carbon or molecular sieve) and the type of essence will depend on the intrinsic characteristics of the essence, on the type and concentration of the solvent in which the essence is dissolved, on the duration in time, on the desired emission speed and will be determined from time to time based on practical experimentation.

Finally, it is important to state that, by using the aforementioned absorbing materials, it is possible to realise inert supports in multiple constructive solutions, such as powders, granules and spheres, as well as cylindrical bars, plates or other manufactures of any solid shape and dimension.

## Claims

1. Inert support for the emission of fragrances, compositions, essences and aromas, **characterised in that** it is made of absorbing material.

2. Inert support for the emission of fragrances, compositions, essences and aromas according to claim 1 **characterised in that** the absorbing material consists in activated carbon in the various levels of activity, expressed as CTC index (carbon tetrachloride) for drawn or extruded cylinders of any size and as iodine index for powder, granules or slices of any size.

3. Inert support for the emission of fragrances, compositions, essences and aromas according to claim 1 **characterised in that** the absorbing material consists in molecular sieve or zeolite, in the various types of specificity measured in Angstrom, both of hydrophilic and hydrophobic type, in any spheroidal, cylindrical shape or powder.

4. Inert support for the emission of fragrances, compositions, essences and aromas according to claim 1 **characterised in that** it has a powder structure.

5. Inert support for the emission of fragrances, compositions, essences and aromas according to claim 1 **characterised in that** it has a granule or sphere structure.

6. Inert support for the emission of fragrances, compositions, essences and aromas according to claim 1 **characterised in that** it has a cylindrical bar structure.

7. Inert support for the emission of fragrances, compositions, essences and aromas according to claim 1 **characterised in that** it has a plate structure.
